Europäisches Patentamt

European Patent Office

Office européen des brevets

(19)

(11) Veröffentlichungsnummer: **0 000 158**
**B1**

(12) **EUROPÄISCHE PATENTSCHRIFT**

(45) Veröffentlichungstag der Patentschrift: **14.10.81**

(21) Anmeldenummer: **78100176.3**

(22) Anmeldetag: **16.06.78**

(51) Int. Cl.³: **C 07 C 51/41,**
C 07 C 55/02, C 07 C 55/14

(54) **Verfahren zur kontinuierlichen Herstellung wässriger Lösungen von Salzen aus Alkandicarbonsäuren und Alkandiaminen.**

(30) Priorität: **27.06.77 DE 2728818**

(43) Veröffentlichungstag der Anmeldung:
**10.01.79 Patentblatt 79/1**

(45) Bekanntmachung des Hinweises auf die Patenterteilung:
**14.10.81 Patentblatt 81/41**

(84) Benannte Vertragsstaaten:
**BE DE FR GB NL**

(56) Entgegenhaltungen:
**DE - A - 2 139 642**
**FR - A - 2 215 411**
**NL - A - 6 507 519**

(73) Patentinhaber: **BASF Aktiengesellschaft**
**Carl-Bosch-Strasse 38**
**D-6700 Ludwigshafen (DE)**

(72) Erfinder: **Rotzoll, Rudi-Heinz, Dr.**
**Donnersbergstrasse 8**
**D-6703 Limburgerhof (DE)**
Erfinder: **Duffner, Paul, Dr.**
**Bozener Strasse 14**
**D-6700 Ludwigshafen (DE)**
Erfinder: **Dietl, Ernst, Dr.**
**Jakobsgarten 7**
**D-6700 Ludwigshafen (DE)**
Erfinder: **Pilz, Georg**
**Ahornweg 4**
**D-6730 Neustadt 19 (DE)**
Erfinder: **Thiel, Gerhard**
**Untergasse 35**
**D-6700 Ludwigshafen (DE)**

Courier Press, Leamington Spa, England.

EP 0 000 158 B1

# Verfahren zur kontinuierlichen Herstellung wäßriger Lösungen von Salzen aus Alkandicarbonsäuren und Alkandiaminen

Gegenstand der Erfindung ist ein Verfahren zur kontinuierlichen Herstellung wäßriger Lösungen von Salzen aus Alkandicarbonsäuren mit 6 bis 12 Kohlenstoffatomen und Alkandiaminen mit 6 bis 12 Kohlenstoffatomen durch Umsetzen der entsprechenden Alkandicarbonsäuren mit den jeweiligen Alkandiaminen in einer wäßrigen Lösung des jeweils herzustellenden Salzes.

Wie aus den bekanntgemachten Unterlagen der NL-Patentanmeldung 65.07519 zu entnehmen ist, werden Salze aus Dicarbonsäuren und Diaminen, z.B. Hexamethylendiammoniumadipat, durch Umsetzen von Dicarbonsäuren und Diaminen im alkoholischen Medium, z.B. Methanol, erzeugt. Das entsprechende Salz fällt aus der Lösung aus und wird durch Zentrifugieren isoliert. Da die Arbeitsweise mit leicht flüchtigen brennbaren Lösungsmitteln in der Technik unerwünscht ist, ist man auch schon dazu übergegangen, solche Salze aus wäßrigen Lösungen zu erzeugen. In der DE—OS 24 03 178 wird ein Verfahren zur Herstellung von festen Salzen aus Alkandicarbonsäuren und Alkandiaminen beschrieben. Hierbei wird feste Dicarbonsäure in einer wäßrigen Lösung aus dem herzustellenden Salz gelöst und diese Lösung in einem Mischbehälter mit dem entsprechenden diese Lösung in einem Mischbehalter mit dem entsprechenden Alkandiamin neutralisiert, wobei sich durch Übersättigung festes Salz ausscheidet. Im Fall der Herstellung von festen Salzen genügt es, beispielsweise einen pH-Wert von 5 bis 10 einzuhalten, da sich im ausscheidenden Salz die äquivalenten Verhältnisse von Dicarbonsäure zu Diaminen von selbst genau einstellen. Die Kristallisation von festem Salz beginnt bereits bevor die Neutralisation vollständig ist. Darüber hinaus ist aus der genannten DE—OS zu entnehmen, daß bei der Neutralisation die Temperatur 80° nicht übersteigen soll. Schließlich wird darauf hingewiesen, daß bei Neutralisation unter Anwendung einer Temperatur von 95—100° unbrauchbare Produkte entstehen. Ein Hinweis, wie hochkonzentrierte Salzlösungen aus Alkandicarbonsäure und Alkandiaminen in kontinuierlicher Arbeitsweise bei Temperaturen über 80°C hergestellt werden und die Einhaltung der Äquivalenz gesichert ist, ist aus der Entgegenhaltung nicht zu entnehmen.

In der Technik ist man allgemein bestrebt, von wäßrigen Lösungen auszugehen, da diese leichter zu handhaben sind. Hierbei ist es jedoch erforderlich, den Äquivalenzpunkt genau einzuhalten, da die selbständige Einstellung durch die Salzausfällung wegfällt. Im Hinblick auf die DE—OS 24 03 178 mußte die anwendung von Temperaturen über 80°C als ungeeignet erscheinen, da nicht brauchbare Salzlösungen zu erwarten waren.

Entsprechend der japanischen Offenlegungsschrift 25 119/72 wird bei der Herstellung von AH-Salz (Hexamethylendiammoniumadipat) zunächst eine wäßrige Lösung von Hexamethylendiamin mit Adipinsäure, vorzugsweise in fester Form, gemischt. Hierbei wendet man das Diamin im Unterschuß an. Die so erhalten Lösung wird dann in einem zweiten großen Mischgefäß auf den erforderlichen pH-Wert eingestellt und mindestens die doppelte Menge des Zulaufs wieder in das erste Mischgefäß zurückgeführt. Wie aus der japanischen Offenlegungsschrift 25 119/72 hervorgeht, dürfen hierbei Temperaturen über 70°C nicht angewandt werden, da sich sonst Verunreinigungen bilden, die Verfärbungen des Polymeran verursachen und auch durch Behandeln mit Aktivkohle nicht mehr beseitigt werden können. Es ist bemerkenswert, daß nach diesen Verfahren die erhaltene wäßrige Lösung auf jeden Fall mit Aktivkohle gereinigt werden muß. Darüber hinaus wird dort ausgeführt, daß es unter Anwendung von wäßrigen Lösungen der Dicarbonsäure nicht gelingt, höher konzentrierte wäßrige AH-Salzlösungen als 32% zu erhalten. Die so erhaltene Lösung muß deshalb vor ihrer Verwendung bei der Polykondensation aufkonzentriert werden. Die Anwendung von fester Adipinsäure für die Herstellung von höher konzentrierten Lösungen ist jedoch insofern nachteilig, als hierbei auch unterhalb 70°C erhebliche Dosierschwierigkeiten auftreten. Die Umsetzung wird entsprechend der japanischen Offenlegungsschrift in einer Rührkesselkaskade mit Rückführung durchgeführt, wobei die Ausgangsstoffe in die Rührkessel eingeführt werden. Hierbei ist ein relativ großes Volumen auf die zugeführte Menge an Ausgangsstoffen erforderlich.

Es war deshalb die technische Aufgabe gestellt, wäßrige Lösungen von Salzen aus Dicarbonsäuren und Diaminen in kontinuierlicher Arbeitsweise so herzustellen, daß äquivalente Verhältnisse eingehalten werden und eine Nachbehandlung nicht erforderlich ist.

Diese Aufgabe wird gelöst in einem Verfahren zur·kontinuierlichen Herstellung von 45 bis 56 gew.-%igen wäßrigen Lösungen von Salzen aus Alkandicarbonsäuren mit 6 bis 12 Kohlenstoffatomen und Alkandiaminen mit 6 bis 12 Kohlenstoffatomen durch Umsetzen der entsprechenden Alkandicarbonsäuren mit den jeweiligen Alkandiaminen in einer wäßrigen Lösung des jeweils herzustellenden Salzes, bei dem man die wäßrige Salzlösung im Kreis führt, zunächst Dicarbonsäure mit Alkandiamin im Unterschuß umsetzt und dann die restliche Menge Alkandiamin zugibt, und wobei man bei einer Temperatur von 90 bis 102°C die wäßrige Salzlösung aus einer ersten Mischzone über eine Förderzone und eine zweite Mischzone in die erste Mischzone zuruckleitet, zwischen der ersten und der zweiten Mischzone flüssiges Alkandiamin und eine wäßrige Lösung von Alkandicarbonsäure zuführt, mit der Maßgabe, daß weniger als die äquivalente Menge an Alkandiamin zugeführt wird, nach der zweiten Mischzone die restliche Menge an flüssigem Alkandiamin zugibt und aus der ersten Mischzone wäßrige Salzlösung in dem Maße entnimmt, wie sie gebildet wird.

Das neue Verfahren hat den Vorteil, daß keine Mutterlaugen anfallen, die behandlungsbedürftig sind. Ferner ist das neue Verfahren sehr flexibel und leicht regelbar. Es eignet sich deshalb in hervorragender Weise für die Ausübung im technischen Maßstab. Das neue Verfahren gestattet die kontinuierliche Herstellung großer Mengen mit kleinem apparativem Aufwand.

Nach dem erfindungsgemäßen Verfahren wird die Umsetzung in einem Mischkreis mit einem Pufferbehälter durchgeführt. Die Ausgangsstoffe werden in die Mischkreisleitung eingeführt. Hierdurch wird bezogen auf die zugeführte Menge an Ausgangsstoffen, ein wesentlich kleineres Reaktionsvolumen ermög-licht und dadurch auch eine wesentlich niedrigere Verweilzeit von z.B. 1 Stunde und weniger erzielt. Die beanspruchte Arbeitsweise gestattet es, die Umsetzung auch bei Temperaturen über 70°C durchzuführen, ohne daß die in der japanischen Offenlegungsschrift 25 119 172 beschriebenen Nachteile eintreten. Es war keineswegs zu erwarten, daß durch die beanspruchte Arbeitsweise die Bildung von Verunreinigungen bei Temperaturen über 70°C vermeiden wird und eine Nachbehandlung nicht mehr erforderlich ist. Im Hinblick auf die in der japanischen Offenlegungsschrift geschilderten Schwierigkeiten war es ein nicht unerhebliches Wagnis, den beanspruchten Weg zu beschreiten. Schließlich gelingt es nach dem bean-spruchten Verfahren, wesentlich konzentrier-tere Lösungen z.B. bis zu 65 Gew.-% herzustellen, die anschließend keiner aufwendigen Aufkonzentration bedürfen.

Als Ausgangsstoffe verwendet man Alkandicarbonsäuren mit 6 bis 12 Kohlenstoffatomen. Bevorzugt geht man von geradkettigen $\alpha$, $\omega$-Alkandicarbonsäuren der genannten Kohlen-stoffzahl aus. Geeignete Dicarbonsäuren sind beispielsweise Adipinsäure, Azelainsäure, Korksäure, Sebacinsäure, Decandisäure oder Dodecandisäure. Besondere technische Bedeutung haben Adipinsäure und Sebacinsäure erlangt.

Ferner verwendet man als Ausgangstoffe Alkandiamine mit 6 bis 12 Kohlenstoffatomen. Bevorzugt geht man von geradkettigen $\alpha$, $\omega$-Alkandiaminen mit der genannten Kohlenstoffzahl aus. Geeignete Alkandiamine sind beispiels weise Hexamethylendiamin, Octamethylendi-amin, Decamethylendiamin oder Dodecan-methylendiamin. Besondere technische Bedeu-tung hat Hexamethylendiamin erlangt.

Demzufolge sind die bevorzugten Salze Hexamethylendiamoniumadipat und Hexa-methylendiammoniumsebacat. Die Konzen-tration der wäßrigen Lösung an den hergestellten Salzen beträgt 45 bis 65, insbesondere 55 bis 65 Gewichtsprozent.

Die jeweils zu verwendende Alkandicarbonsäure wird mit einem entsprechenden Alkandiamin in einer wäßrigen Lösung des jeweils herzustellenden Salzes umgesetzt. Es versteht sich, daß die Konzentration der verwendeten wäßrigen Lösung des Salzes derjenigen des Erzeugnisses entspricht.

Die wäßrige Salzlösung wird aus einer ersten Mischzone über eine Förderzone une eine zweite Mischzone in die erste Mischzone zurückgeleitet. Vorteilhaft beträgt die in der ersten Mischzone befindliche Menge an Salz-lösung das 2- bis 3-fache gegenüber der in sämtlichen übrigen Zonen und Leitungen befindlichen Menge an Salzlösung. In der Regal verwendet man als erste Mischzone einen Rührkessel oder eine adäquate Vorrichtung mit Mischorganen, z.B. Kreislaufpumpen, wobei darauf zu achten ist, daß die erste Mischzone eine ausreichende Pufferkapazität gegenüber den übrigen Zonen aufweist. Als Förderzone verwendet man kontinuierlich fördernde Pumpen, wie z.B. Kreiselpumpen. Die zweite Mischzone ist vorteilhaft als Mischstrecke ausgebildet, d.h. man sorgt durch Umlenkungen und/oder Einbauten für eine rasche wirkungsvolle Durchmischung des durchströmenden Mediums. Vorteilhaft wird stündlich die 40- bis 80-fache Menge des Inhalts der ersten Mischzone im Kreis geführt.

Zwischen der ersten Mischzone und der zweiten Mischzone wird Alkandiamin in flüssiger Form und Alkandicarbonsäure als wäßrige Lösung zugeführt. Die Alkandiamine werden vorteilhaft in geschmolzener Form angewandt. Es ist jedoch auch möglich, Alkandiamine durch Zugabe von geringen Mengen an Wasser, z.B. bis zu 20 Gewichtsprozent zu verflüssigen. Alkandicarbonsäuren werden vorteilhaft in 48 bis 55 gewichtsprozentiger wäßriger Lösung zugeführt. Es versteht sich, daß die insgesamt zugeführte Menge an Wasser so bemessen sein muß, daß nach Vereinigung eine der gewünschten Konzentration entsprechende Lösung von Salzen aus Alkandicarbonsäuren und Alkandiaminen resultiert. Die Menge der zugeführten Alkandiamine wird so gewählt, daß sie unterhalb der nötigen äquivalenten Menge, bezogen auf die Menge an Dicarbonsäure, liegt. Vorteilhaft führt man 95 bis 99 Molprozent der zu Äquivalenz nötigen Diaminmenge zu.

Vorteilhalft führt man zunächst Alkandiamine zu insbesondere auf der Saugseite der Förderzone und dann die wäßrige Lösung an Alkandicarbonsäuren, vorzugsweise auf der Druckseite der Förderzone.

Das Reaktionsgemisch, das nun eine geringe Menge an überschüssiger Alkandicarbonsäure enthält, wird durch eine zweite Mischzone

geleitet, um eine Salzbildung zu gewährleisten. Anschließend an die zweite Mischzone wird die restliche Menge an flüssigem Alkandiamin zugeführt, die zur Erreichung des Äquivalenzpunktes erforderlich ist. Der Äquivalenzpunkt läßt sich für die einzelnen Salze leicht anhand des pH-Wertes bestimmen. Er beträgt für Hexamethylendiammoniumadipat 7,65 (gemessen als 10%-ige wäßrige Lösung bei 25°C) und für Hexamethylendiammoniumsebacat 7,60. Es ist auch möglich Alkandiamin in geringem Überschuß z.B. bis zu 0.5 Molprozent zuzusetzen und Verluste an Alkandiamin bei der Kondensation auszugleichen. Vorteilhaft folgt auf die Zugabe der restlichen Menge an Alkandiamin eine weitere Mischzone, die in ihrem Aufbau der zweiten Mischzone entspricht. Ex hat sich bewährt, wenn die stündlich insgesamt zugeführte Menge an Ausgangsstoffen, d.h. Alkandiaminen, Alkandicarbonsäuren und Wasser dem 0,5 bis 2-fachen der im gesamten Kreislauf befindlichen Menge an wäßriger Salzlösung entspricht.

Die erhaltene wäßrige Lösung von Salzen aus Alkandicarbonsäuren und Alkandiaminen wird wieder in die erste Mischzone zurückgeführt. Vorteilhaft wird die wäßrige Lösung vorher durch einen Kühler geleitet. Bei der Umsetzung hält man eine Temperatur von 90 bis 102°C ein.

Besonders vorteilhaft ist es, wenn man die Zugabe der restlichen Menge an Alkandiaminen aufgrund des pH-Wertes der im Kreis geführten wäßrigen Salzlösung steuert. Dies erfolgt z.B. so, daß man nach der Zugabe der vorgenannten Ausgangsstoffe fortlaufend eine kleine Menge an Salzlösung entnimmt, diese auf 10% verdünnt, den pH-Wert mißt und aufgrund des gemessenen pH-Werts die Zugabemenge an Alkandiamin nach der zweiten Mischzone steuert. Vorteilhaft ist es wenn man zur Steuerung eine verdünnte Alkandiaminlösung, z.B. bis zu 25% anwendet.

Die Umsetzung wird in der Regel bei Atmosphärendruck oder schwach erhögtem Druck durchgeführt. In der ersten Mischzone hält man vorteilhaft eine Inertgasatmosphäre, z.B. Stickstoffatmosphäre, aufrecht. Aus der ersten Mischzone wird die wäßrige Salzlösung in dem Maße entnommen, wie sie entsteht, z.B. durch einen Überlauf.

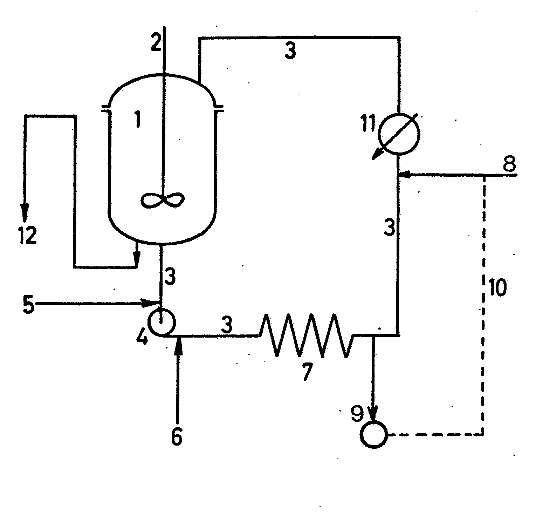

Das Verfahren nach der Erfindung wird beispielsweise an Figur 1 erläutert. In ein Kreislaufsystem, bestehend aus einem Rührkessel 1 mit einem Rührer 2, einem Kreislauf 3, einer Förderpumpe 4, einer Mischstrecke 7 und einem Kühler 11 wird auf der Saugseite der Pumpe 4 über Leitung 5 flüssiges Alkandiamin zugeführt, während man auf der Druckseite der Pumpe über die Leitung 6 eine wäßrige Lösung der Alkandicarbonsäure zuführt. Über die Leitung 8 wird die restliche Menge an flüssigem Alkandiamin zugeführt. Mittels Leitung 9 entnimmt man fortlaufend einen kleinen Teil der wäßrigen Salzlösung und verdünnt sie auf 10% und mißt den pH-Wert. Die ermittelten Werte werden als Impuls über die Meßleitung 10 auf die Zuführungsleitung 8 übertragen und so die Zulaufmenge geregelt. Die entstehende Salzlösung wird mittels der Überlaufleitung 12 dem System in dem Maße entnommen, wie sich die Lösung bildet.

Wäßrige Lösungen von Salzen aus Alkandicarbonsäuren und Alkandiaminen werden zur Herstellung von Polyamiden verwendet.

Das Verfahren nach der Erfindung sei an folgendem Beispiel veranschaulicht.

### Beispiel

Einem Kreislaufsystem, bestehend aus einem 5 m³ Rührkessel mit Rührer, einer Kreislaufpumpe einer durch Umlenkorgane gebildeten Mischstrecke, einem Kühler und einer pH-Regelung werden stündlich 3,8 m³ 52,5%-ige Adipinsäure-Lösung und 1.65 t geschmolzenes Hexamethylendiamin zugeführt. Es wird nur soviel Neutralisationswärme abgeführt, um das System auf 98°C zu halten. Mit einer Kreislaufpumpe werden 250 m³/h AH-Salz-Lösung im Kreis umgepumpt. Stündlich werden 10 kg/h AH-Salz-Lösung entnommen und mit Wasser auf 10% AH-Salz-Gehalt verdünnt. Über den pH-Wert dieser Lösung erfolgt die Steuerung der Zugabe von 10%-igem Hexamethylendiamin, welches zum Erreichen der Äquivalenz von pH 7,65 noch nötig ist. Über einen standhaltenden Syphon fließen stündlich 5,95 m³ 63%-ige AH-Salz-Lösung aus dem Kreislaufsystem ab.

### Patentansprüche

1. Verfahren zur kontinuierlichen Herstellung von 45 bis 65 gew.-%igen wäßrigen Lösungen von Salzen aus Alkandicarbonsäuren mit 6 bis 12 Kohlenstoffatomen und Alkandiaminen mit 6 bis 12 Kohlenstoffatom durch Umsetzen der entsprechenden Alkandicarbonsäuren mit den jeweiligen Alkandiaminen in einer wäßrigen Lösung des jeweils herzustellenden Salze, bei dem man die wäßrige Salzlösung im Kreis führt, zunächst Dicarbonsäure mit Alkandiamin im Unterschuß umsetzt und dann die restliche Menge Alkandiamin zugibt, dadurch gekennzeichnet, daß man bei einer Temperatur von 90 bis 102°C die wäßrige Salzlösung aus einer ersten Mischzone über eine Förderzone und eine zweite Mischzone in die erste Mischzone zurückleitet, zwischen der ersten und der zweiten Mischzone flüssiges Alkandiamin und eine wäßrige Lösung von Alkandicarbonsäure zuführt, mit der Maßgabe, daß weniger als die äquivalente Menge an Alkandiamin zugeführt wird, nach der zweiten Mischzone die restliche Menge an flüssigem Alkandiamin zugibt und aus der ersten Mischzone wäßrige Salzlösung in dem Maße entnimmt, wie sie gebildet wird.

2. Verfahren nach Anspruch 1, dadurch gekennzeichnet, daß man auf der Saugseite der Förderzone flüssiges Alkandiamin zuführt.

3. Verfahren nach Anspruch 1, dadurch gekennzeichnet, daß man auf der Druckseite der Förderzone die wäßrige Lösung an Alkandicarbonsäure zuführt.

4. Verfahren nach Anspruch 1, dadurch gekennzeichnet, daß die Zugabe der restlichen Menge an flüssigem Alkandiamin durch den pH-Wert der wäßrigen Salzlösung vor dem Wiedereintritt in die erste Mischzone gesteuert wird.

5. Verfahren nach Anspruch 1, dadurch gekennzeichnet, daß man die 40- bis 80-fache Menge an der in der ersten Mischzone befindlichen Salzlösung stündlich umwälzt.

## Claims

1. A process for the continuous preparation of an aqueous solution, of from 45 to 65% strength by weight, of a salt of an alkanedicarboxylic acid of 6 to 12 carbon atoms by reacting the particular alkanedicarboxylic acid with the particular alkanediamine in an aqueous solution of the salt to be prepared, the aqueous salt solution being recycled, dicarboxylic acid being first reacted with a less than equivalent amount of alkanediamine, and the remaining amount of alkanediamine then being added, characterized in that the aqueous salt solution is recycled at a temperature of from 90 to 102°C from a first mixing zone via a transport zone and a second mixing zone into the first mixing zone, liquid alkanediamine and an aqueous solution of alkanedicarboxylic acid are introduced between the first and second mixing zones, with the proviso that less than the equivalent amount of alkanediamine is introduced, the remaining amount of liquid alkanediamine is added after the second mixing zone, and aqueous salt solution is taken off the first mixing zone at the rate at which it is formed.

2. A process as claimed in claim 1, characterized in that liquid alkanediamine is introduced on the intake side of the transport zone.

3. A process as claimed in claim 1, characterized in that the aqueous solution of alkanedicarboxylic acid is introduced on the delivery side of the transport zone.

4. A process as claimed in claim 1, characterized in that the addition of the remaining amount of liquid alkanediamine is controlled by the pH of the aqueous salt solution before re-entry into the first mixing zone.

5. A process as claimed in claim 1, characterized in that from 40 to 80 times the amount contained in the first mixing zone of salt solution is circulated per hour.

## Revendications

1. Procédé de préparation en continu de solutions aqueuses à 45 à 65% de sels d'acides alcanedicarboxyliques ayant 6 à 12 atomes de carbone et d'alcanediamines ayant 6 à 12 atomes de carbone, par réaction des acides alcanedicarboxyliques correspondants avec les alcanediamines dans une solution aqueuse du sel à préparer, procédé dans lequel on recycle la solution de sel aqueuse, on fait réagir ensuite des acides dicarboxyliques avec de l'alcanediamine en quantité insuffisante et on ajoute les quantités restantes d'alcanediamine, procédé caractérisé par le fait que, à une température de 90 à 102°C, on ramène à une première zone de mélange, en passant par une zone d'alimentation et une deuxième zone de mélange, la solution de sel aqueuse provenant de ladite première zone de mélange, on amène, entre la première et la deuxième zone de mélange, de l'alcanediamine liquide et une solution aqueuse d'acide alcanedicarboxylique, sous réserve que l'on amène moins que la quantité équivalente d'alcanediamine, on ajoute après la deuxième zone de mélange, la quantité restante d'alcanediamine liquide et on prélève de la première zone de la solution de sel aqueuse dans la mesure où ils ont été formés.

2. Procédé selon la revendication 1, caractérisé par le fait que l'on amène de l'alcanediamine liquide au côté aspiration de la zone d'alimentation.

3. Procédé selon la revendication 1, caractérisé par le fait que l'on amène la solution aqueuse d'acide alcanedicarboxylique au côté refoulement de la zone d'alimentation.

4. Procédé selon la revendication 1, caractérisé par le fait que l'on commande l'addition de la quantité restante d'alcanediamine liquide par la valeur du pH de la solution saline aqueuse avant le retour dans la première zone de mélange.

5. Procédé selon la revendication 1, caractérisé par le fait que l'on recycle par heure une quantité égale à 40 à 80 fois la solution saline qui se trouve dans la première zone de mélange.